(19) Europäisches Patentamt
European Patent Office
Office européen des brevets

(11) Publication number: 0 158 000 B1

# (12) EUROPEAN PATENT SPECIFICATION

(45) Date of publication of patent specification: 19.06.91 (51) Int. Cl.5: C07D 233/70, A01N 43/50

(21) Application number: 85100111.5

(22) Date of filing: 07.01.85

## (54) Regio and optical isomers of imidazolinyl toluic acids, esters and salts, their method of preparation and their use as herbicidal agents.

(30) Priority: 10.02.84 US 579224

(43) Date of publication of application:
16.10.85 Bulletin 85/42

(45) Publication of the grant of the patent:
19.06.91 Bulletin 91/25

(84) Designated Contracting States:
AT BE CH DE FR GB IT LI LU NL SE

(56) References cited:
EP-A- 0 095 105
FR-A- 2 400 018
US-A- 4 188 487

(73) Proprietor: AMERICAN CYANAMID COMPANY
1937 West Main Street P.O. Box 60
Stamford Connecticut 06904-0060(US)

(72) Inventor: Los, Marinus
107 Drummond Drive
Pennington, NJ 08534(US)

(74) Representative: Wächtershäuser, Günter, Dr.
Tal 29
W-8000 München 2(DE)

EP 0 158 000 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

The present invention relates to imidazolinyl toluic acids, esters and salts represented below by formula (I). More precisely, the invention relates to the dextrorotatory optical isomers of the meta and para regioisomers as well as the racemic mixture of the meta and para regioisomers.

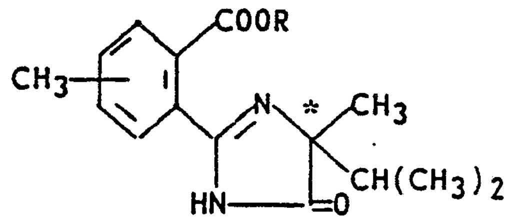

formula (I)

wherein R is hydrogen, methyl, a salt-forming cation of an alkali metal, ammonium or aliphatic ammonium, and the aromatic methyl group is meta and/or para to the -COOR-substituent; and except when R is a salt-forming cation, the acid-addition salts thereof. The asterisk in formula (I) above indicates the chiral center.

The invention also relates to a heretofor unavailable method for the preparation of essentially pure individual formula (I) regio- and/or optical isomers of imidazolinyl toluates.

The invention further relates to a method for selectively and more efficiently controlling undesirable plant species with the various regio- and optical isomers of said imidazolinyl toluates, said method comprising, applying to the foliage of the undesirable plant species or to the soil containing seeds, seedlings or propagating organs of the undesirable plant species, a herbicidally-effective amount of the appropriate regio-and/or optical isomer of said formula (I) imidazolinyl toluate.

The preferred formula (I) compound is the (+) optical isomer, and the meta-toluic acid, either as the racemic (±) mixture or as the (+) optical isomer; and the para-toluic acid, either as the racemic mixture or as the (+) optical isomer; wherein R is methyl or a salt-forming cation of alkali metals, ammonium and aliphatic ammonium.

Although certain regio and optical isomeric mixtures of herbicidal imidazolinyl benzoates are described in U.S. Patent No. 4,188,487, issued February 12, 1980, this reference fails to disclose a practical method for preparing each of the regio and optical isomers of said toluic acids. Also, the reference does not recognize the varying herbicidal selectivity and effectiveness of the individual regio and optical isomers of the formula (I) toluic acids, esters and salts.

The regioisomer mixtures of U. S. Patent 4,188,487 are obtained during preparation. For instance, if aromatic substitutions are present, cyclization of the phthalimidocarboxamide gives rise to a 50/50 mixture of both imidazoisoindolediones because cyclization may occur at either imide carbonyl group (see Figure I below). This mixture is not easily separated and in order to give rise to the mixture of imidazolyl toluates, it must be reacted directly with methanol and sodium methoxide as described in U. S. Patent No. 4,188,487.

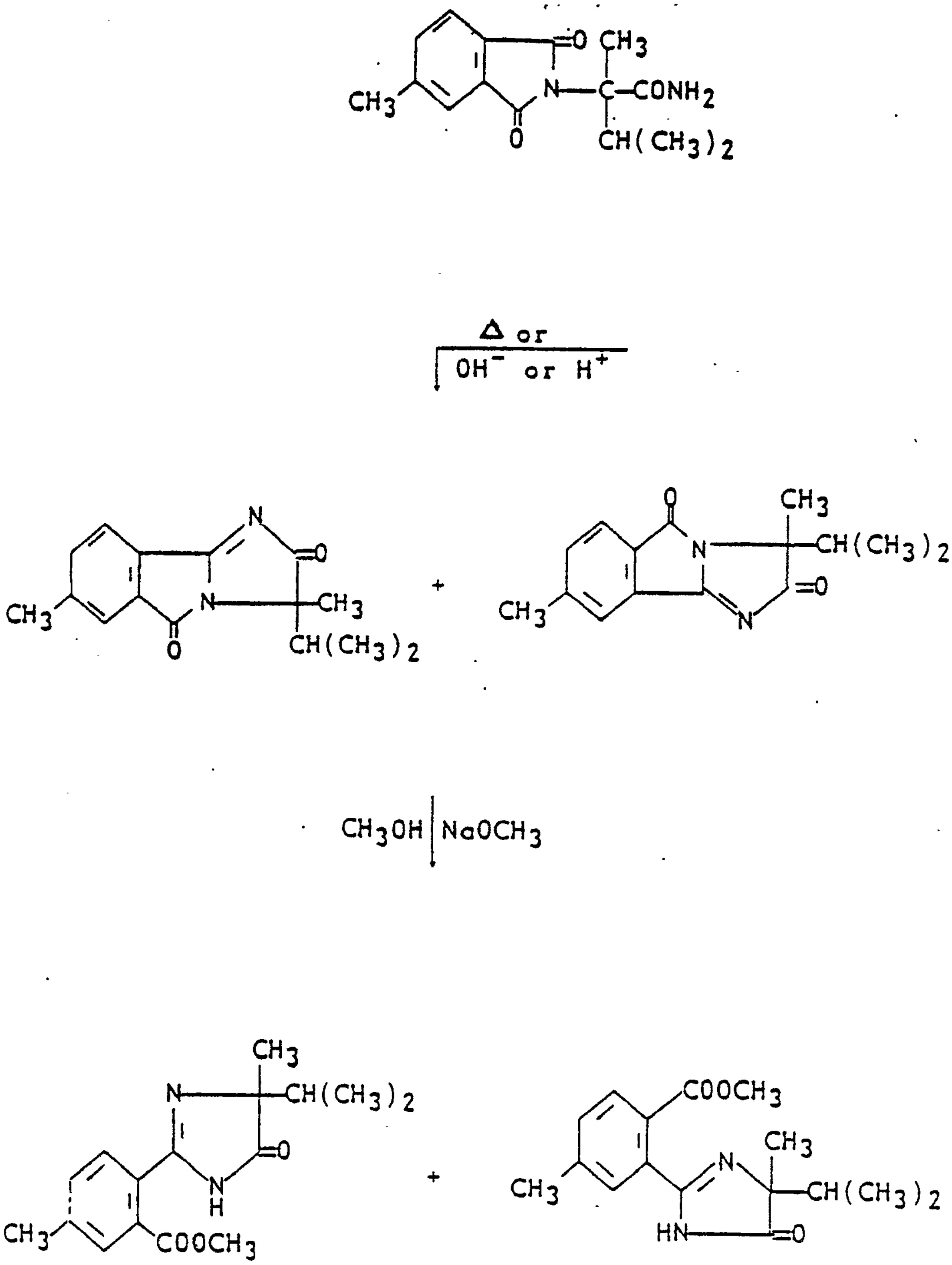


FIGURE I

As such, there is no practical method for the isolation of both the pure meta- and para-methyl toluates.

It is an object of the present invention therefore to provide satisfactory methods for preparing each of the essentially pure regio and optical isomers of the formula I compounds.

Further, it is an object of this invention to provide such compounds having superior herbicidal activity as well as a spectrum of herbicidal activity (selectivity).

The present invention provides a compound selected from the group consisting of:

methyl (+)-2-(4-isopropyl-4-methyl-5-oxo-2-imidazolin-2-yl)-p-toluate, having a rotation of about $[alpha]_D^{25}$ +1.06° (C = 0.026 g/ml THF) and a melting point of 138° C-140° C; methyl (+)-6-(4-isopropyl-4-methyl-5-oxo-2-imidazolin-2-yl)-m-toluate, having a rotation of about $[alpha]_D^{25}$ +3.29° (C = 0.029 g/ml THF) and a melting point of 137°-140° C; (+)-2-(4-isopropyl-4-methyl-5-oxo-2-imidazolin-2-yl)-p-toluic acid, having a rotation of about $[alpha]_D^{25}$ +6.09° (C = 0.027 g/ml THF); (+)-6-(4-isopropyl-4-methyl-5-oxo-2-imidazolin-2-yl)-m-toluic acid, having a rotation of about $[alpha]_D^{25}$ +5.37° (C = 0.042 g/ml THF); (±)-2-(4-isopropyl-4-methyl-5-oxo-2-imidazolin-2-yl)-p-toluic acid, (±)-6-(4-isopropyl-4-methyl-5-oxo-2-imidazolin-2-yl)-m-toluic acid, having a melting point of 185°-187° C; or the acid addition salt thereof; or the alkali metal, ammonium or aliphatic ammonium salt thereof.

It has been found that the individual meta- and para-toluates of the invention may be prepared satisfactorily from meta- and para-toluic acids as indicated below.

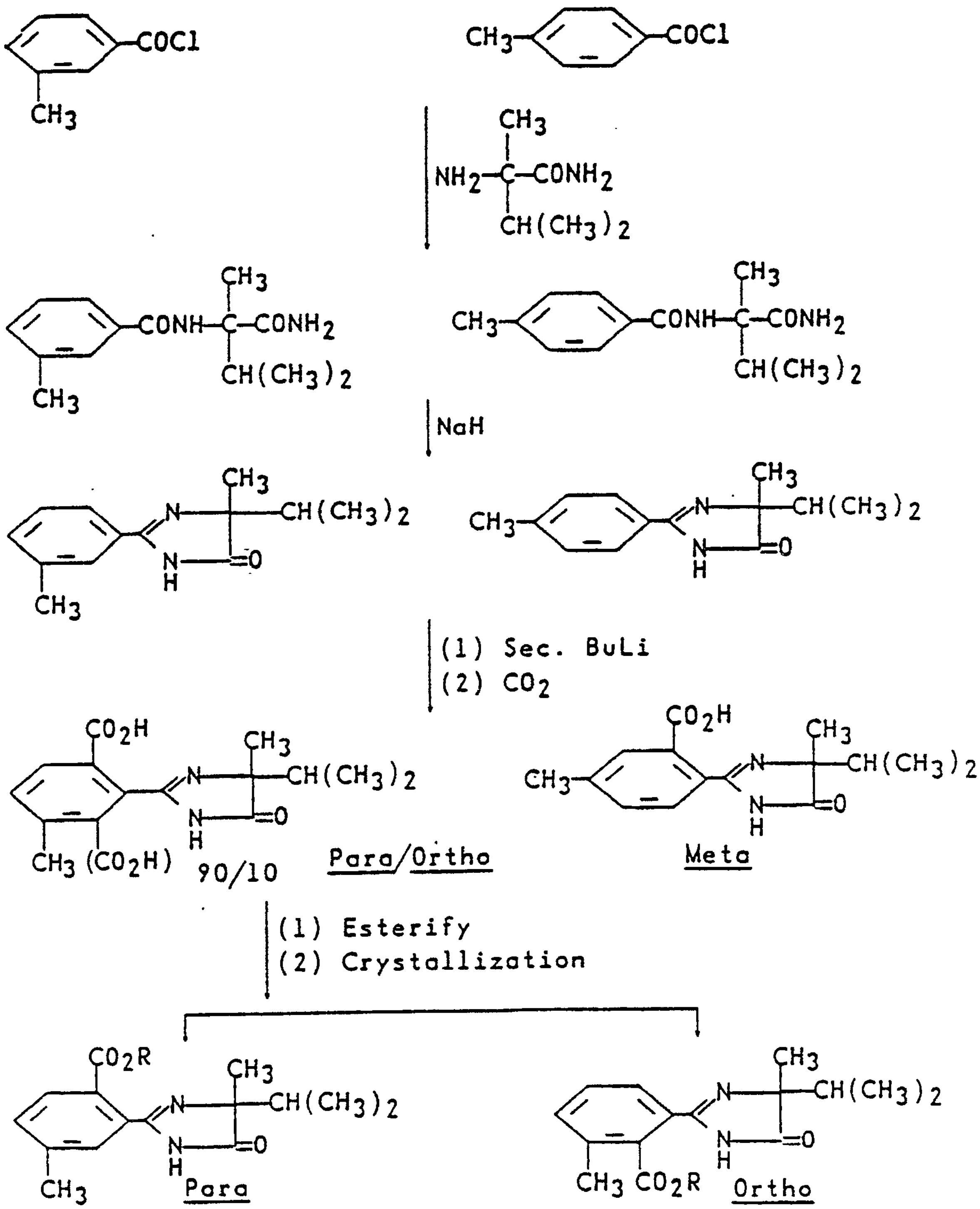


FIGURE 2

The para-toluic acid yields the meta-imidazolinyl toluic acid, whereas, by a similar process, the meta-toluic acid gives a 90/10 mixture of para/ortho imidazolinyl toluic acids which, after conversion to their methyl esters, are readily separated by crystallization and/or chromatography. Optically pure compounds of each of the regioisomers or a mixture of the regioisomers may be obtained by using optically pure 2-amino-2,3-dimethylbutyramide in the above sequences.

Comparison of the individual optical isomers of the regio-isomer mixture (meta/para ~50/50), shows that the (-)-optical isomer has very little herbicidal activity at 4 kg/ha, while the (+)-isomer is, on the average,

1.8 times more active on weeds than the racemic mixture, while being as safe on the crops as the racemic mixture.

The para-imidazolinyl toluates as the racemic mixture and the (+)-optical isomer thereof exhibit herbicidal activity on the broadest spectrum of weeds, with the racemic mixture having an average control rate of 1.6 kg/ha and the pure (+)-optical isomer having an average control rate of 0.5 kg/ha, while still maintaining essentially the same safety for cereal crops as the regio and optical isomer mixture.

Although the racemic or (+)-isomer of the meta-imidazolinyl toluates do not seem to control Green foxtail (S. vividis), or Wild mustard (B. kaber) at 4 kg/ha, they are two to four times more active than the para-isomers on Black grass (A. myosuroide) and Wild oats (A. fatua). The (+)-isomer of the meta-imidazolinyl toluate is also found to be twice as active as the racemic compound on these weeds whilst both compounds show the same selectivity on cereals. These data are summarized in Table I.

It should also be understood that the imidazolinyl toluates represented by formula (I) above may be tautomeric. While, for convenience, they are depicted by a single structure identified as formula (I), they may exist in either of the tautomeric forms illustrated in Figure 3:

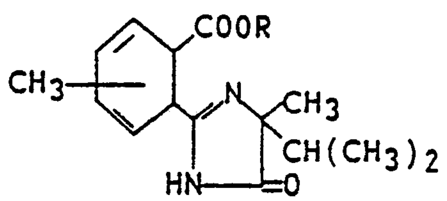

or

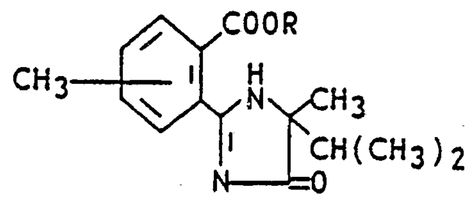

FIGURE 3

As such, both tautomeric forms of the imidazolinyl toluates are included under the formula (I) definition.

These compounds are amphoteric. They will dissolve in both acidic and basic aqueous solutions, and when treated with strong acids, particularly strong mineral acids such as hydrochloric acid, sulfuric acid or hydrobromic acid, they will form the acid addition salts of the imidazolinyl toluates.

The compounds of this invention are highly effective herbicidal agents useful for the control of both monocotyledonous, sedge (cyperacious) and dicotyledonous plants. They may be employed for the post-emergence control of undesirable plant species by applying a herbicidally-effective amount thereof to the foliage of the plants, or they may be used for the pre-emergence control of the undesirable plants by applying a herbicidally-effective amount of the active compound to soil containing seeds, seedlings or propagating organs of the undesirable plants.

Compositions

Since the imidazolinyl toluates (I) of the present invention exhibit very limited solubility in water, they are generally formulated as wettable powders, emulsifiable concentrates or flowable liquids which are usually dispersed in water or other inexpensive liquid diluent for application as a liquid spray. The compounds of the invention may also be prepared as granular formulations containing generally about 10 to 15% by weight of toxicant.

Typically, a wettable powder can be prepared by grinding together about 25% to 80% by weight of the imidazolinyl toluate, about 2% to 5% by weight of a surfactant such as sodium N-methyl-N-oleoyl taurate, alkyl phenoxy polyoxyethylene ethanol, or sodium alkyl naphthalene sulfonate, about 5% to 10% by weight of a dispersing agent such as a highly purified sodium lignosulfonate, and 25% to 63% by weight of a finely divided carrier such as kaolin, attapulgite, diatomaceous earth or the like.

A typical formulation prepared in accordance with the above description is as follows:
50% by weight of (+)-methyl-2-(4-isopropyl-4-methyl-5-oxo-2-imidazolin-2-yl)-p-toluate, 3% by weight of sodium N-methyl-N-oleoyl taurate, 10% by weight of sodium lignosulfonate and 37% by weight of kaolin.

Flowable liquid formulations can be prepared by grinding together about 40% to 60% by weight of formula (I) imidazolinyl toluate, about 2% to 3% by weight of the sodium salt of condensed naphthalene sulfonic acid, about 2% to 3% by weight of a gelling clay, about 2% by weight of propylene glycol and about 54% to 32% by weight of water.

A typical granular formulation can be prepared by dissolving or dispersing the active compound in a

solvent and applying the toxicant to a sorptive or non-sorptive carrier such as attapulgite, corncob grits, pumice, talc or the like.

As indicated above, the imidazolinyl compounds, depicted by formula (I) are effective preemergence herbicides. They are highly effective for the control of broadleaf weeds and grass plants when applied at a rate of from about 0.10 kg per hectare to 11.2 kg per hectare to soil containing seeds, seedlings or propagating organs of the broadleaf weeds, sedges or grass plants.

The compounds of the invention are also effective for the control of broadleaf weeds, sedges, and grass plants when applied at the rate of from about 0.28 kg per hectare to 11.2 kg per hectare to the foliage of the plants.

While the compounds of this invention are very effective for controlling a wide variety of plant species, they are unique among herbicides in their ability to control certain cyperacieous plants, particularly sedges, at relatively low rates of application. In practice, the formula (I) compounds have been found to be most effective as sedge control agents when applied as a preemergence application at rates of from 0.14 kg per hectare to 11.2 kg per hectare. It is of course, recognized that higher rates of application of the formula (I) compounds can be used for sedge and other perennial plant control when infestations of the cyperacae or perennial plants are especially heavy. Under such conditions, the formula (I) imidazolinyl benzoates may be applied, preemergence or postemergence, at rates as high as 25 kg per hectare.

Among the cyperacae which can be controlled with the imidazolinyl benzoates of this invention are purple nutsedge (Cyperus rotundus L.) a yellow nutsedge (Cyperus esulentus L.) , false nutsedge (Cyperus strigosus) and the flat sedges, umbrella plants and kyllinga.

This invention is further illustrated by the following examples:

EXAMPLE 1 (Reference)

**Resolution of 2-amino-2,3-dimethylbutyronitrile**

To an ice-cooled solution of 80 g (0.53 mol), (+)-tartaric acid in 200 ml water is added 56 g (0.50 mol), of (±)-2-amino-2,3-dimethylbutyronitrile. During the addition, the mixture is kept cold. The precipitate is collected by filtration, washed with a little ice-cold water and air dried to give 65.25 g of the tartrate salt of (+)-2-amino-2,3-dimethylbutyronitrile.

The filtrate isolated from the tartrate salt is treated with `56 ml concentrated ammonia and extracted three times with ether. The combined extracts are washed with brine, dried and concentrated to give 24 g (-)-2-amino-2,3-dimethylbutyronitrile with $[\alpha]_D^{25}$ = -4.49° (C = 0.0376 g/ml THF).

The (-)-aminonitrile is converted again to the salt with 39 g (-)-tartaric acid in 54 ml water, the salt collected, and the aminonitrile liberated from the salt with 26 ml concentrated ammonium hydroxide as described above. This process gives 19.3 g of (-)-2-amino--2,3-dimethylbutyronitrile with $[\alpha]_D^{25}$ = -5.89° (C = 0.0353 g/ml THF).

Two further cycles of salt preparation and liberation of the amino nitrile with ammonia give 11.8 g of (-)-2-amino-2,3-dimethylbutyronitrile with $[\alpha]_D^{25}$ = -7.31° (C = 0.0368 g/ml THF).

When salt derived from (+)-tartaric acid and (+)-2-amino-2,3-dimethylbutyronitrile is put through the same sequence of liberation and salt formation with (+)-tartaric acid two times, the (+)-2-amino-2,3-dimethylbutyronitrile obtained has $[\alpha]_D^{25}$ = +6.93° (C = 0.085 g/ml THF).

EXAMPLE 2 (Reference)

**Preparation of 2-amino-2,3-dimethylbutyramide**

Concentrated sulfuric acid (29.7 ml) is cooled with stirring in an ice-acetone cooling bath. To the acid is added 11.8 g (011 mol) (-)-2-amino-2,3-dimethylbutyronitrile with $[\alpha]_D^{25}$ = -7.31° (C = 0.0368 g/ml THF) at such a rate that the temperature does not go above 25° C. After the addition, the temperature of the reaction mixture is slowly raised to 100° C and held at that temperature for one hour. After cooling the mixture in an ice-acetone bath, 85 ml concentrated ammonia is added at such a rate that the temperature does not exceed 25° C. The mixture is extracted five times with methylene chloride, the combined extracts dried and concentrated, giving 11.95 g of white solid, mp 79° C-81° C and $[\alpha]_D^{25}$ = +57.43° (C = 0.0213 g/ml THF). This solid is recrystallized from methylene chloride-hexane to give 11.2 g of (+)-2-amino-2,3-dimethylbutyramide, mp 81° C-82° C and $[\alpha]_D^{25}$ = +59.38° (C = 0.0162 g/ml THF).

In a similar way, hydrolysis of the (+)-2-amino--2,3-dimethylbutyronitrile with sulfuric acid yields the (-)-2-amino-2,3-dimethylbutyramide, mp 81° C-82° C, $[\alpha]_D^{25}$ = -57.14° (C = 0.0654 g/ml THF).

Hydrolysis of the (±)-2-amino-2,3-dimethylbutyronitrile with sulfuric acid yields (±)-2-amino-2,3-dimethylbutyramide, mp 74.5° C-76° C.

EXAMPLE 3 (Reference)

**Preparation of (-)-6-[(1-carbamoyl-1,2-dimethylpropyl)carbamoyl]-m-toluic acid and (-)-2-[1-carbamoyl-1,2-dimethylpropyl)carbamoyl]-p-toluic acid**

To a solution containing 15 g (0.12 mol) of (+)-2,3-dimethylbutyramide in 150 ml dry THF under nitrogen is added 18.7 g (0.12 mol) 4-methylphthalic anhydride. The mixture is then stirred at room temperature overnight. Thin layer chromatographic analysis indicates complete reaction. The mixture is concentrated to give the product, a mixture of the 4- and 5-methylphthalamic acids, as a glass which is characterized by its NMR spectrum and used without further purification. This crude product has a negative rotation.

EXAMPLE 4

**Preparation of (+)-6-(4-isopropyl-4-methyl-5-oxo-2-imidazolin-2-yl)-m-toluic acid and (+)-2-(4-isopropyl-4-methyl-5-oxo-2-imidazolin-2-yl)-p-toluic acid**

The crude product mixture from Example 3, (35.07 g) is dissolved in 23.8 ml of 50% aqueous sodium hydroxide and warmed at 85° C for two hours. After cooling to 0° C, the pH of the solution is adjusted to pH 3 with 6N $H_2SO_4$. The mixture is extracted with methylene chloride, the extract dried and concentrated to give the crude product (34.06 g) as a glass with IR and NMR spectra consistent with that expected for the product.

This same mixture of (+)-6-(4-isopropyl-4-methyl-5-oxo-2-imidazolin-2-yl)-m-toluic acid and (+)-2-(4-isopropyl-4-methyl-5-oxo-2-imidazolin-2-yl)-p-toluic acid is obtained when the corresponding methyl ester mixture of Example 5 is hydrolyzed with 2N NaOH. The product is obtained as an amorphous solid with $[\alpha]_D^{25}$ = +4.3° (C = 0.0813 g/ml THF).

To 1.7 g (6.2 mmol) of this same mixture in 50 ml absolute methanol is added 0.8 ml (8.6 mmol) isopropylamine. After stirring at room temperature overnight, the mixture is concentrated in vacuo to give an amorphous solid. This is dissolved in methylene chloride dried by $Na_2SO_4$, filtered and concentrated to give 1.42 g of amorphous solid. After thorough drying, this material has a NMR spectrum consistent with the assigned structure. This material has $[\alpha]_D^{25}$ = +13.6° (C = 0.051 g/ml THF).

Other ammonium salts can be prepared in a similar fashion. The inorganic salts are prepared by reacting the organic acid with one equivalent of an alkali metal hydroxide, oxide or carbonate in aqueous methanol and removing the solvent to give the desired salt which is purified by conventional means.

EXAMPLE 5

**Preparation of methyl (+)-2-(4-isopropyl-4-methyl-5-oxo-2-imidazolin-2-yl)-p-toluate and methyl (+)-6-(4-isopropyl-4-methyl-5-oxo-2-imidazolin-2-yl)-m-toluate**

To 24.8 g (0.12 mol) dicyclohexylcarbodiimide in 200 ml dry $CH_2Cl_2$ is added the crude acid prepared in Example 4. The reaction is exothermic. After stirring at room temperature for two hours, the mixture is filtered and the filtrate concentrated. The residue is dissolved in 200 ml methanol, and 0.5 g sodium methoxide is added. The mixture is stirred overnight followed by heating at reflux for one hour. Enough acetic acid is then added to the solution to indicate a pH of 4 as measured by moist pH paper. The mixture is concentrated, the residue dissolved in $CH_2Cl_2$ and the solution washed with saturated sodium bicarbonate solution. The organic phase is then washed with saturated brine, dried and concentrated to give 34 g crude product. Chromatography of this material on silica gel using 1:1 ether-hexane followed by ether as eluent gives the pure product which is crystallized from ether-hexane, mp 115° C-121.5° C, $[\alpha]_D^{25}$ = +0.55° (C = 0.1254 g/ml THF). Analysis of this product by HPLC shows it to be a 54:46 mixture of methyl (+)-2-(4-isopropyl-4-methyl-5-oxo-2-imidazolin-2-yl)-p--toluate and methyl (+)-6-(4-isopropyl-4-methyl-5-oxo-2-imidazolin-2-yl)-m-toluate.

EXAMPLE 6 (Reference)

**Preparation of (±)-N-(1-carbamoyl-1,2-dimethylpropyl)-m-toluamide**

To a solution containing 26 g (0.20 mol), (±)-2-amino-2,3-dimethylbutyramide and 30.7 ml trimethylamine in 200 ml dry tetrahydrofuran at 0° c is added dropwise 26.4 ml (0.20 mol) m-toluoyl chloride. The temperature is kept below 15° C. After the addition, stirring is continued for one hour at room temperature. The mixture is poured into 300 ml ice-cold water. The product is collected by filtration, washed with water and air dried to give 52.3 g of crude products. A 4.0 g sample is recrystallized from methylene chloride-hexane to give 2.58 g of analytically pure (±)-N-(1-carbamoyl-1,2-dimethylpropyl)-m-toluamide, mp 186.5-187.5° C. When (+)-2-amino-2,3-dimethylbutyramide is used in place of the racemic material, the product is (-)-N-(1-carbamoyl-1,2-dimethylpropyl)-m-toluamide, mp 164° C-179° C, $[\alpha]_D^{25}$ = -39.32° (C = 0.0509 g/ml methanol).

EXAMPLE 7 (Reference)

**Preparation of (±)-4-isopropyl-4-methyl-2-m-tolyl-2-imidazolin-5-one**

A suspension of sodium hydride (19.56 g of a 50% suspension in mineral oil (0.40 mol)) in 200 ml toluene is heated at reflux under a Dean-Stark water separator. The (±)-N-(1-carbamoyl-1,2-dimethylpropyl)-m -toluamide (46.0 g, (0.185 mol)) is added portionwise to this boiling suspension. Heating is continued for 20 minutes after the addition. The mixture is cooled, 23.5 ml glacial acetic acid added slowly followed by 150 ml water. The aqueous phase is separated, the pH adjusted to 4.5 with 6N sulfuric acid and extracted twice with 200 ml methylene chloride. The original toluene layer is combined with the methylene chloride extracts, dried and concentrated. Titration of the residue with ether-hexane gives 31 g of crude product. Recrystallization of 10 g of this material from ether-hexane gave 9.8 g analytically pure (±)-4-isopropyl-4-methyl-2-m-tolyl-2-imidazolin-5-one, mp 102.5°-105° C.

When the reaction is carried out on the product from (+)-2-amino-2,3-dimethylbutyramide, there is obtained (+)-4-isopropyl-4-methyl-2-m-tolyl-imidazolin-5-one, mp 94°-97° C, $[\alpha]_D^{25}$ = +0.36° (C = 0.0272 g/ml THF).

EXAMPLE 8

**Preparation of (+)-2-(4-isopropyl-4-methyl-5-oxo-2-imidazolin-2-yl)-p-toluic acid**

A stirred solution containing 20 g (0.087 mol) of (+)-4-isopropyl-4-methyl-2-m-tolyl-2-imidazolin-5-one under dry nitrogen in 580 ml dry tetrahydrofuran is cooled to -78° C. To this solution is added, dropwise, 212.3 ml (0.191 mol, 2.2 equivalent) of sec-butyl lithium in cyclohexane. The maximum temperature during the addition is -60° C. After the addition, the mixture is allowed to warm to -25° C and held at that temperature for one hour. The mixture is then transferred gradually to a flask containing a stirred slurry of solid carbon dioxide in a liter of dry tetrahydrofuran. After stirring overnight, the solvent is removed in vacuo, the residue dissolved in 500 ml water and this solution extracted with methylene chloride. The organic phase is discarded. The pH of the aqueous phase is adjusted to pH 3 with 6N sulfuric acid, and this phase is extracted with 3 x 200 ml methylene chloride. The combined extracts are dried and concentrated to give a mixture of the two acids as a glass in a ratio of about 9:1 p-toluic to o-toluic acid. This material was used directly for the preparation of the corresponding methyl esters.

The pure p-toluic acid can be obtained by hydrolysis of the corresponding methyl ester (of Example 9) with sodium hydroxide to give the product 6-(4-isopropyl-4-methyl-5-oxo-2-imidazolin-2-yl)-p-toluic acid as an amorphous solid with $[\alpha]_D^{25}$ = +6.09° (C = 0.0266 g/ml THF).

EXAMPLE 9

**Preparation of (+)-methyl 2-(4-isopropyl-4-methyl-5-oxo-2-imidazolin-2-yl)-p-toluate**

The mixture of acids (21.24 g) prepared as described in Example 8 and derived from the (+)-amino amide dissolved in 100 ml methylene chloride is added dropwise to a stirred solution cooled to 0° C and containing 15.97 g (0.078 mol) dicyclohexylcarbodiimide. After the addition, the mixture is stirred at room temperature overnight. The mixture is filtered and 2 ml glacial acetic acid added to the filtrate. The filtrate is concentrated in vacuo and filtered as necessary to remove precipitates. Residual acetic acid is removed by dissolving the residue in xylene and concentrating the solution. This is repeated. The residue is dissolved in

200 ml methanol; 1.8 g sodium methoxide is added, and the mixture is stirred at room temperature for ten minutes. Thin layer chromatography shows the reaction to be complete. After the addition of 2 ml glacial acetic acid, the mixture is concentrated in vacuo, the residue dissolved in methylene chloride and the organic phase washed with saturated aqueous sodium bicarbonate. The organic phase is separated, dried and concentrated to give an oily residue. Trituration with ether gives a crystalline solid. This is recrystallized from methylene chloride-hexane, mp 138°-140°, $[\alpha]_D^{25}$ = +1.06° (C = 0.0264 g/ml THF). This material is (+)-methyl 2-(4-isopropyl-4-methyl-5-oxo-2-imidazolin-2-yl)-p-toluate.

EXAMPLE 10 (Reference)

**Preparation of (±)-N-(1-carbamoyl-1,2-dimethylpropyl)-p-toluamide**

To a stirred solution containing 10.0 g (0.077 mol) of (±)-2-amino-2,3-dimethylbutyramide and 12 ml triethylamine in 125 ml acetone is added, dropwise, 11.9 g (0.077 mol) p-toluoyl chloride. After the addition, the mixture is stirred a further hour at room temperature. Then the mixture is poured into 300 ml water with stirring. The white solid is removed by filtration, washed with water and air dried to give 15.8 g product mp 154°-159°C. This is recrystallized from acetonitrile to give 11.6 g of analytically pure (±)-N-(1-carbamoyl-1,2-dimethyl-propyl)-p-toluamide mp 160°-162°C.

When the reaction is repeated using the (+)-2-amino-2,3-dimethylbutyramide rather than the (±)-2-amino-2,3-dimethylbutyramide, the corresponding (-)-N-(1-carbamoyl-1,2-dimethylpropyl)-p-toluamide is obtained, mp 138°-140°C, $[\alpha]_D^{25}$ = -57.18° (C = 0.0288 g/ml THF).

EXAMPLE 11 (Reference)

**(±)-4-Isopropyl-4-methyl-2-p-tolyl-2-imidazolin-5-one**

To 125 ml dry toluene is added 2.7 g (0.056 mol), of a 50% suspension of sodium hydride in mineral oil. The mixture is stirred, heated under reflux, and 7.0 g (0.028 mol), of (±)-N-(1-carbamoyl-1,2-dimethylpropyl)-p-toluamide is added portionwise at this temperature. After heating overnight, 10 ml isopropyl alcohol is added to the cooled mixture, and the mixture is poured into 200 ml ice-water. The aqueous phase is separated, washed with toluene and then acidified with glacial acetic acid to a pH of 4. The resulting oil slowly solidifies and is removed by filtration, washed with water and air dried to give 5.6 g product mp 148°-151°C. This is recrystallized from acetonitrile to give 4.9 g of analytically pure (+)-4-isopropyl-4-methyl-2-p-tolyl-2-imidazolin-5-one mp 151°C-154°C.

When the reaction is carried out on (-)-N-(1-carbamoyl-1,2-dimethylpropyl)-p-toluamide, then the product is (-)-4-isopropyl-4-methyl-2-p-tolyl-2-imidazolin-5-one mp 181°-182°C, $[\alpha]_D^{25}$ = 6.1° (C = 0.0382 g/ml THF).

EXAMPLE 12

**Preparation of (±)-6-(4-isopropyl-4-methyl-5-oxo-2-imidazolin-2-yl)-m-toluic acid**

To a stirred solution containing 15.1 g (0.065 mol), of (±)-5-isopropyl-5-methyl-2-p-tolyl-2-imidazolin-4-one in 400 ml dry tetrahydrofuran under nitrogen is added at -55°C, 125 ml of a 1.17M solution of sec-butyl lithium (2.2 equivalents) in cyclohexane. After the addition, the mixture is maintained at a temperature of -20 to -40°C for three hours. The mixture is then added, while stirring, to a slurry of solid carbon dioxide in about one liter of tetrahydrofuran. After stirring overnight, the mixture is concentrated and the residue dissolved in a mixture of ether and water. The ether phase is separated, washed with water and the aqueous phase separated and combined with the first aqueous phase. The aqueous phase is acidified to pH 3-4 with 6N sulfuric acid and extracted several times with methylene chloride. The organic extracts are dried (sodium sulfate) and concentrated. Crystallization from methylene chloride hexane gives (±)-6-(4-isopropyl-4-methyl-5-oxo-2-imidazolin-2-yl)-m-toluic acid, mp 185°C-187°C.

When the above reaction is carried out with (-)-4-isopropyl-4-methyl-2-p-tolyl-2-imidazolin-5-one the product, (+)-6-(4-isopropyl-4-methyl-5-oxo-2-imidazolin-2-yl)-m-toluic acid is obtained as an amorphous solid with $[\alpha]_D^{25}$ = +5.37° (C = 0.0418 g/ml THF).

EXAMPLE 13

**Preparation of (+)-methyl 6-(4-isopropyl-4-methyl-5-oxo-2-imidazolin-2-yl)-m-toluate**

To an ice-cold solution containing 23.97 g (0.087 mol), of the acid in 200 ml methylene chloride is added a slight excess of etherial diazomethane. The excess diazomethane is destroyed with acetic acid and the mixture concentrated in vacuo. The residue is crystallized from ether to give (+)-methyl 6-(4-isopropyl-4-methyl-5-oxo-2-imidazolin-2-yl)-m-toluate which, when recrystallized from methylene chloride-hexane has mp 137°C-140°C, $[\alpha]_D^{25}$ = +3.29° (C = 0.0297 g/ml THF).

EXAMPLE 14

**Postemergence herbicidal activity and selectivity of the regio and optical isomers of imidazolinyl toluates**

The postemergence herbicidal activity and selectivity of the compounds of the invention is demonstrated by the following experiments:

Seeds of the test species are planted in the upper 1.25 cm of soil (loamy sand) contained in either 6.35 $cm^2$ plastic pots or 100 $cm^2$ fiber pots. For the tests, the plants are allowed to grow in the greenhouse for two to three weeks before treatment. After treatment, the plants are returned to the greenhouse for an additional six to eight weeks, at which time the herbicidal activity is evaluated.

All herbicide applications are made with a belt sprayer set to deliver 374 l/ha. The compounds are suspended in 80:20 acetone:$H_2O$ which contains a non-ionic surfactant for post application. All treatments are replicated two to five times.

Results of the herbicide evaluation are expressed on a rating scale of zero to nine. Based on this scale, a rating ≧8 is considered a control rating for weed species, while a safe rating on a crop is ≦2. The results of these tests are summarized in Table I.

**Plant Abbreviations**

| | |
|---|---|
| Black Grass | *Alopercurus myosurcides* |
| Green Fox | *Seturia vividis* |
| Wild Oats | *Avena fatua* |
| Wild Mustard | *Brassica kaber* |
| Barley MA | *Hordeum vulgare*, CV Maury |
| Barley BE | *Hordeum vulgare*, CV Beacon |
| Wheat AZ | *Triticum aestivum*, CV Anza |
| Wheat NU | *Triticum aestivum*, CV Nugaines |

TABLE I

Comparison of the optical and positional isomers of methyl imidazolinyl toluates

Postemergence test - rates in kg/ha

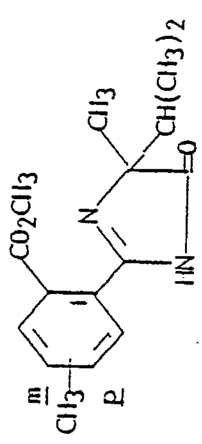

| Optical Isomer | Regio Isomer | Controls at | | | | Safe at | | | |
|---|---|---|---|---|---|---|---|---|---|
| | | Black Grass | Green Fox | Wild Oats | Wild Mustd | W Barley MA | S Barley Be | S Wheat Az | W Wheat Nu |
| (±)* | m/p ~50/50 | 0.60 | 2.00 | 0.60 | 0.40 | 4.00 | 4.00 | 4.00 | 4.00 |
| (+) | m/p ~50/50 | 0.30 | 2.00 | 0.30 | 0.30 | 2.00 | 2.00 | 2.00 | 2.00 |
| (±)* | p | 2.00 | 2.00 | 2.00 | 0.30 | 4.00 | 4.00 | 4.00 | 4.00 |
| (+) | p | 0.50 | 0.70 | 0.70 | 0.10 | 4.00 | 3.00 | 3.00 | 4.00 |
| (±)* | m | 0.60 | >4.00 | 0.40 | >4.00 | 4.00 | 4.00 | 4.00 | 4.00 |
| (+) | m | 0.30 | >4.00 | 0.20 | >4.00 | 4.00 | 4.00 | 4.00 | 4.00 |

*Control

As can be seen, the (+) optical isomers of the imidazolinyl toluate exhibit superior herbicidal activity on weeds while remaining soft on crop species.

The para imidazolinyl toluates racemic mixture exhibited the broadest herbicidal activity, with the racemic mixture having an average control rate of 1.6 kg/ha, with the pure (+) optical isomer having an average control rate of 0.5 kg/ha.

EXAMPLE 15

**Postemergence herbicidal activity**

The postemergence herbicidal activity of the compounds of the invention is demonstrated by the following tests, wherein a variety of monocotyledonous, cyperaceous and dicotyledonous plants are treated with test compounds dispersed in aqueous acetone mixtures. In the tests, seedling plants are grown in separate cups for 50/50 acetone/water mixtures containing 0.5% TWEEN® 20, a polyoxyethylene sorbitan monolaurate surfactant of Atlas Chemical Industries, in sufficient quantity to provide the equivalent of about 0.07 kg to 11.2 kg per hectare of active compound when applied to the plants through a spray nozzle operating at 2.8 kg/$Cu^2$ pressure for a predetermined time. After spraying, the plants are placed on greenhouse benches and are cared for in the usual manner, commensurate with conventional greenhouse practices. Two weeks after treatment, the seedling plants, with the exception of wild oats, which are rated at five weeks, are examined and rated according to the rating system provided below. The data obtained are reported in Table II below, which contains averaged data from several tests.

| Rating | Meaning | % Control |
|---|---|---|
| 9 | Complete kill | 100 |
| 8 | Approaching complete kill | 91-99 |
| 7 | Good herbicidal effect | 80-90 |
| 6 | Herbicidal effect | 65-79 |
| 5 | Definite injury | 45-64 |
| 4 | Injury | 30-44 |
| 3 | Moderate effect | 16-29 |
| 2 | Slight effect | 6-15 |
| 1 | Trace effect | 1-5 |
| 0 | No effect | 0 |
| P | "PGR" effect | - |
| X | Unable to read sample | - |

Plant Abbreviations

| Abbreviations | Common Name | Scientific Name |
|---|---|---|
| Barnyardgr | Barnyardgrass | Echinochloa crus-galli, (L) Beau |
| Blackgrass | Blackgrass | Alopecurus myosuroides |
| Canarygras | Canarygrass spp. | Phalaris spp. (L) |
| Cheat | Cheat | Bromus secalinus, L. |
| Crabgrass | Crabgrass spp. | Digitaria spp. |
| Green fox | Foxtail, Green | Setaria viridis, (L) Beauv |
| Lolium spp | Lolium Spp. | Lolium spp. |
| Wild oats | Oat, wild | Avena fatua, L. |
| Lolium per | Lolium | Lolium Perenne, L. |
| Fld Bindwd | Bindweed, Field | Convolvulus arvensis, |
| Lambsqtrs | Lambsquarters, Common | Chenopodium album, L. |
| Mrnglry sp | Morningglory spp. | Ipomoea spp. |
| Wild mustd | Mustard, wild | Brassica kaber, (DC) L.C. Wheelr |

Table 11

Postemergence herbicidal activity for compounds having the structure

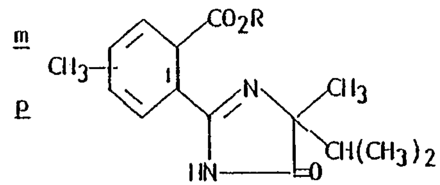

| R | Optical isomer (±) | Regio isomer meta/para | Rate kg per hectare | BARNY ARDGR | BLACK GRASS | CATHAR YGRAS | CHEAT | CRABG RASS | GREEN FOX | LOLIU M SPP | WILD OATS | LOLIU M PER | FLD B INDWD | LAMBS QTRS | MRNGL RY SP | WILD MUSTD |
|---|---|---|---|---|---|---|---|---|---|---|---|---|---|---|---|---|
| | | | | Plant Species | | | | | | | | | | | | |
| $CH_3$ | (±)* | m/p ~50/50 | 4.000 | 7.5 | 9.0 | 5.9 | 4.3 | 0.0 | 7.0 | 6.0 | 8.9 | 0.0 | 9.0 | 6.5 | 7.0 | 8.8 |
| | | | 2.000 | 5.8 | 8.6 | 6.4 | 3.5 | 0.3 | 5.6 | 3.2 | 8.8 | 2.4 | 8.3 | 4.0 | 4.3 | 8.9 |
| | | | 1.000 | 1.1 | 8.3 | 5.5 | 1.8 | 0.0 | 3.0 | 1.5 | 8.6 | 0.4 | 7.5 | 1.8 | 3.7 | 8.7 |
| | | | .500 | 0.2 | 5.5 | 2.6 | 0.3 | 0.0 | 1.7 | 0.2 | 7.7 | 0.0 | 5.8 | 1.6 | 2.0 | 7.3 |
| $CH_3$ | (±)* | p | 4.000 | 8.0 | 8.9 | 3.0 | 0.0 | 0.0 | 9.0 | | 9.0 | 0.0 | 9.0 | 5.0 | 5.0 | 9.0 |
| | | | 2.000 | 1.0 | 7.6 | 0.0 | 0.0 | 0.0 | 5.6 | | 8.6 | 0.0 | 9.0 | 1.0 | 1.0 | 8.8 |
| | | | 1.000 | 0.0 | 5.3 | 0.0 | 0.0 | 0.0 | 5.1 | | 8.1 | 0.0 | 9.0 | 0.0 | 0.0 | 8.7 |
| | | | .500 | 0.0 | 2.1 | 0.0 | 0.0 | 0.0 | 2.8 | | 4.3 | 0.0 | 9.0 | 0.0 | 0.0 | 8.3 |
| $CH_3$ | (±)* | m | 4.000 | 5.0 | 9.0 | 9.0 | 0.0 | 9.0 | 4.9 | | 9.0 | 0.0 | 8.0 | 7.0 | 5.0 | 6.4 |
| | | | 2.000 | 1.0 | 8.7 | 9.0 | 0.0 | 1.0 | 2.7 | | 9.0 | 0.0 | 7.0 | 0.0 | 1.0 | 0.7 |
| | | | 1.000 | 0.0 | 8.2 | 7.0 | 0.0 | 0.0 | 2.3 | | 8.7 | 0.0 | 1.0 | 0.0 | 0.0 | 0.0 |
| | | | .500 | 0.0 | 7.6 | 1.0 | 0.0 | 0.0 | 0.3 | | 8.3 | 0.0 | 0.0 | 0.0 | 0.0 | 0.0 |
| $CH_3$ | (+) | m/p ~50/50 | 4.000 | | 9.0 | 0.0 | 7.0 | | 8.5 | | 9.0 | 7.0 | | 7.0 | | 9.0 |
| | | | 2.000 | 8.7 | 9.0 | 8.5 | 6.7 | 0.0 | 6.6 | 9.0 | 9.0 | 7.0 | | 6.0 | | 9.0 |
| | | | 1.000 | 5.3 | 8.3 | 8.0 | 3.9 | 0.0 | 5.4 | 8.0 | 9.0 | 2.0 | | 5.0 | | 9.0 |
| | | | .500 | 1.7 | 7.5 | 6.5 | 0.2 | 0.0 | 3.7 | 3.7 | 8.9 | 0.0 | | 0.0 | | 8.8 |

*Control

Table II (Continued)

| R | Optical isomer (±) | Regio isomer meta/para | Rate kg per hectare | Plant Species BARNY ARDGR | BLACK GRASS | CANAR YGRAS | CHEAT | CRABG RASS | GREEN FOX | WILD OATS | LOLIU M PER | FLD B INDWD | LAMBS QTRS | MRNGL RY SP | WILD MUSTD |
|---|---|---|---|---|---|---|---|---|---|---|---|---|---|---|---|
| H | (±) | m | 2.000 | 5.0 | 7.0 | 2.0 | 0.0 | 0.0 | 9.0 | 9.0 | 0.0 | 9.0 | 0.0 | 5.0 | 9.0 |
| | | | 1.000 | 5.0 | 6.0 | 1.0 | 0.0 | 0.0 | 5.0 | 9.0 | 0.0 | 9.0 | 0.0 | 1.0 | 5.0 |
| | | | .500 | 4.0 | 0.0 | 0.0 | 0.0 | 0.0 | 4.0 | 9.0 | 0.0 | 9.0 | 0.0 | 0.0 | 1.0 |
| | | | .250 | 3.0 | 0.0 | 0.0 | 0.0 | 0.0 | 1.0 | 1.0 | 0.0 | 5.0 | 0.0 | 0.0 | 0.0 |
| H | (±) | p | 2.000 | 1.0 | 0.0 | 0.0 | 0.0 | | 8.0 | 5.0 | 0.0 | 9.0 | 1.0 | 1.0 | 9.0 |
| | | | 1.000 | 0.0 | 0.0 | 0.0 | 0.0 | | 8.0 | 1.0 | 0.0 | 9.0 | 0.0 | 0.0 | 9.0 |
| | | | .500 | 0.0 | 0.0 | 0.0 | 0.0 | | 3.0 | 0.0 | 0.0 | 9.0 | 0.0 | 0.0 | 9.0 |
| | | | .250 | 0.0 | 0.0 | 0.0 | 0.0 | | 0.0 | 0.0 | 0.0 | 9.0 | 0.0 | 0.0 | 5.0 |

EXAMPLE 16

**Preemergence herbicidal activity**

The preemergence herbicidal activity of the compounds of the present invention is exemplified by the following tests in which the seeds or propagating organs of a variety of monocotyledonous, cyperaccous and dicotyledonous plants are separately mixed with potting soil and planted on top of approximately 25 cu of soil in separate (size) cups. After planting, the cups are sprayed with the selected aqueous acetone solution containing test compounds in sufficient quantity to provide the equivalent of about 0.28 kg to 11.2 kg per hectare of test compound per cup. The treated cups are then placed on greenhouse benches, watered and cared for in accordance with conventional greenhouse procedures. Three or four weeks after treatment, the test are terminated and each cup is examined and rated according to the rating system set forth in Example 6. The herbicidal proficiency of the active ingredient of the present invention is evident from the test results which are reported in Table III below.

Table III

Preemergence herbicidal activity for compounds having the structure

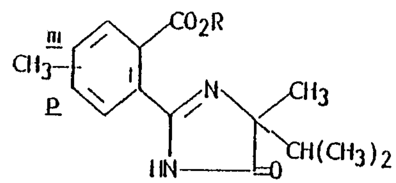

| R | Optical isomer (±) | Regio isomer meta/para | Rate kg per hectare | BARLY ABDCR | BLACK GRASS | CAHAR YGRAS | CHEAT | CRABG RASS | GREEN FOX | LOLIU M SPP | WILD OATS | LOLIU M FER | FLD B INDWD | LAMBS QTRS | MRNGL RY SP | WILD MUSTD |
|---|---|---|---|---|---|---|---|---|---|---|---|---|---|---|---|---|
| $CH_3$ | (±)* | m/p ~ 50/50 | 4.000 | 9.0 | 9.0 | 9.0 | 9.0 | 8.0 | 9.0 | 8.0 | 9.0 | 8.0 | 9.0 | 9.0 | 8.3 | 9.0 |
| | | | 2.000 | 7.9 | 8.8 | 9.0 | 6.3 | 6.5 | 8.5 | 6.0 | 8.9 | 7.4 | 9.0 | 6.9 | 7.0 | 8.3 |
| | | | 1.000 | 6.6 | 8.8 | 8.4 | 4.7 | 4.0 | 7.4 | 5.0 | 8.5 | 4.4 | 9.0 | 6.3 | 5.8 | 8.1 |
| | | | .500 | 3.0 | 7.2 | 6.0 | 2.7 | 2.5 | 4.9 | 4.0 | 8.0 | 1.6 | 9.0 | 4.4 | 3.4 | 7.7 |
| $CH_3$ | (±)* | p | 4.000 | 9.0 | 9.0 | 9.0 | 9.0 | 9.0 | 9.0 | | 9.0 | 9.0 | 9.0 | 9.0 | 8.0 | 9.0 |
| | | | 2.000 | 9.0 | 9.0 | 9.0 | 9.0 | 8.0 | 9.0 | | 8.5 | 9.0 | 9.0 | 7.0 | 8.0 | 8.0 |
| | | | 1.030 | 8.0 | 9.0 | 9.0 | 8.0 | 7.0 | 8.5 | | 8.0 | 9.0 | 9.0 | 4.0 | 8.0 | 7.0 |
| | | | .500 | 6.0 | 9.0 | 9.0 | 7.0 | 2.0 | 7.5 | | 6.5 | 7.0 | 9.0 | 2.5 | 7.0 | 6.0 |
| $CH_3$ | (±)* | m | 4.000 | 9.0 | 9.0 | 9.0 | 9.0 | 8.0 | 8.0 | | 0.0 | 9.0 | 9.0 | 9.0 | 9.0 | 9.0 |
| | | | 2.000 | 7.0 | 9.0 | 9.0 | 9.0 | 7.0 | 8.0 | | 0.0 | 9.0 | 9.0 | 8.0 | 8.0 | 9.0 |
| | | | 1.000 | 1.0 | 9.0 | 9.0 | 8.0 | 2.0 | 5.0 | | 0.0 | 8.0 | 9.0 | 7.0 | 1.0 | 9.0 |
| | | | .500 | 0.0 | 9.0 | 8.0 | 7.0 | 1.0 | 4.0 | | 0.0 | 5.0 | 9.0 | 5.0 | 1.0 | 7.0 |
| $CH_3$ | (+) | m/p ~ 50/50 | 4.000 | | | 9.0 | 9.0 | | 9.0 | | 9.0 | 9.0 | | 9.0 | | 9.0 |
| | | | 2.000 | | | 9.0 | 9.0 | | 8.0 | | 9.0 | 7.0 | | 8.0 | | 9.0 |
| | | | 1.000 | | | 8.0 | 7.0 | | 8.0 | | 9.0 | 5.0 | | 7.0 | | 9.0 |
| | | | .500 | | | 7.0 | 3.0 | | 5.0 | | 8.0 | 3.0 | | 5.0 | | 8.0 |

*Control

Table III (Continued)

| R | Optical isomer (±) | Regio isomer meta/para | Rate kg per hectare | Plant Species | | | | | | | | | | | |
|---|---|---|---|---|---|---|---|---|---|---|---|---|---|---|---|
| | | | | BARLY ARDGR | BLACK GRASS | CANAR YGRAS | CHEAT | CRABG RASS | GREEN FOX | WILD OATS | LOLIU M PER | FLD B INDWD | LAMBS QTRS | MRNGL RY SP | WILD MUSTD |
| H | (±) | m | 2.000 | 9.0 | 9.0 | 9.0 | 9.0 | 9.0 | 9.0 | 9.0 | 9.0 | 9.0 | 9.0 | 9.0 | 8.0 |
| | | | 1.000 | 9.0 | 9.0 | 9.0 | 9.0 | 8.0 | 8.0 | 9.0 | 9.0 | 9.0 | 9.0 | 9.0 | 9.0 |
| | | | .500 | 9.0 | 9.0 | 9.0 | 9.0 | 8.0 | 8.0 | 9.0 | 9.0 | 9.0 | 9.0 | 9.0 | 8.0 |
| | | | .250 | 9.0 | 9.0 | 8.0 | 9.0 | 7.0 | 7.0 | 8.0 | 9.0 | 9.0 | 8.0 | 9.0 | 8.0 |
| H | (±) | p | 2.000 | 9.0 | 9.0 | 9.0 | 9.0 | 9.0 | 9.0 | 9.0 | 9.0 | 9.0 | 9.0 | 8.0 | 9.0 |
| | | | 1.000 | 8.0 | 9.0 | 9.0 | 9.0 | 8.0 | 8.0 | 8.0 | 9.0 | 9.0 | 9.0 | 8.0 | 9.0 |
| | | | .500 | 5.0 | 9.0 | 9.0 | 9.0 | 8.0 | 7.0 | 8.0 | 9.0 | 9.0 | 8.0 | 8.0 | 9.0 |
| | | | .250 | 2.0 | 9.0 | 8.0 | 9.0 | 5.0 | 5.0 | 7.0 | 8.0 | 9.0 | 7.0 | 7.0 | 9.0 |

## Claims

1. A compound selected from the group consisting of: methyl (+)-2-(4-isopropyl-4-methyl-5-oxo-2-imidazolin-2-yl)-p-toluate, having a rotation of about $[alpha]_D^{25}$ +1.06° (C = 0.026 g/ml THF) and a

melting point of 138° C-140° C; methyl (+)-6-(4-isopropyl-4-methyl-5-oxo-2-imidazolin-2-yl)-m-toluate, having a rotation of about $[alpha]_D^{25}$ +3.29° (C = 0.029 g/ml THF) and a melting point of 137°-140° C; (+)-2-(4-isopropyl-4-methyl-5-oxo-2-imidazolin-2-yl)-p-toluic acid, having a rotation of about $[alpha]_D^{25}$ +6.09° (C = 0.027 g/ml THF); (+)-6-(4-isopropyl-4-methyl-5-oxo-2-imidazolin-2-yl)-m-toluic acid, having a rotation of about $[alpha]_D^{25}$ +5.37° (C = 0.042 g/ml THF); (±)-2-(4-isopropyl-4-methyl-5-oxo-2-imidazolin-2-yl)-p-toluic acid; (±)-6-(4-isopropyl-4-methyl-5-oxo-2-imidazolin-2-yl)-m-toluic acid having a melting point of 185°-187° C; addition salt thereof; or the alkali metal, ammonium or aliphatic ammonium salt thereof.

2. A method for the control of undesirable plant species which comprises applying to the foliage of the plants or to soil containing seeds, seedlings or propagating organs of the plants, a herbicidally-effective amount of a compound selected from the group consisting of: methyl (+)-2-(4-isopropyl-4-methyl-5-oxo-2-imidazolin-2-yl)-p-toluate, having a rotation of about $[alpha]_D^{25}$ +1.06° (C = 0.026 g/ml THF) and having a melting point of 138°-140° C; methyl (+)-6-(4-isopropyl-4-methyl-5-oxo-2-imidazolin-2-yl)-m-toluate, having a rotation of about $[alpha]_D^{25}$ +3.29° (C = 0.029 g/ml THF) and a melting point of 137°-140° C; (+)-2-(4-isopropyl-4-methyl-5-oxo-2-imidazolin-2-yl)-p-toluic acid, having a rotation of about $[alpha]_D^{25}$ +6.09° (C = 0.027 g/ml THF); (+)-6-(4-isopropyl-4-methyl-5-oxo-2-imidazolin-2-yl)-m-toluic acid, having a rotation of about $[alpha]_D^{25}$ +5.37° (C = 0.042 g/ml THF); (±)-2-(4-isopropyl-4-methyl-5-oxo-2-imidazolin-2-yl)-p-toluic acid; (±)-6-(4-isopropyl-4-methyl-5-oxo-2-imidazolin-2-yl)-m-toluic acid; or the acid addition salt thereof; or the alkali metal, ammonium or aliphatic ammonium salt thereof.

3. A process for the preparation of the dextrorotatory and racemic mixtures of each of the meta and para regioisomers of compounds of the structural formula,

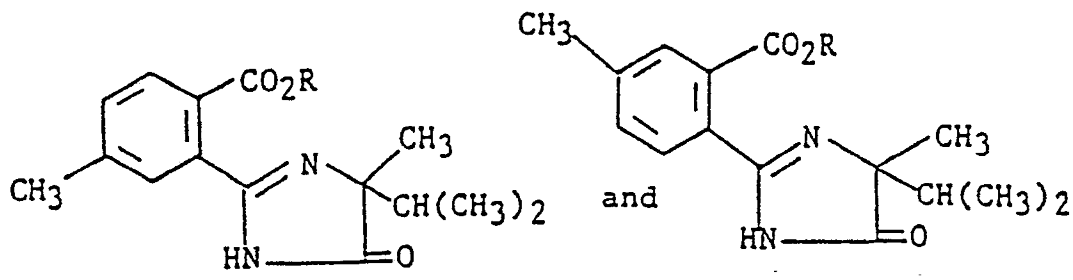

and

wherein R is H, said process characterized by: reacting either p-toluoyl chloride or m-toluoyl chloride with one equivalent of racemic or (+)-2-amino-2,3-dimethylbutyramide to form the N-(1-carbamoyl-1,2-dimethylpropyl)toluamide; cyclizing said toluamide with two equivalents of a strong base, such as sodium hydride, to yield a 4-isopropyl-4-methyl-2-tolyl-2-imidazolin-5-one; metallating said 2-imidazolin-5-one with 2.2 equivalents sec-butyl lithium; and treating the thus-formed reaction mixture with $CO_2$ to give the desire toluic acid.

4. The process according to Claim 3, wherein said toluoyl chloride is the meta toluoylchloride; said 2,3-dimethylbutyramide is the racemic 2-amino-2,3-dimethylbutyramide; and said compound is (±)-2-(4-isopropyl-4-methyl-5-oxo-2-imidazolin-2-yl)-p-toluic acid; wherein said toluoyl chloride is the meta toluoyl chloride; said 2-amino-2,3-dimethylbutyramide is (+); and said compound is (+)-2-(4-isopropyl-4-methyl-5-oxo-2-imidazolin-2-yl)-p-toluic acid; wherein said toluoyl chloride is para toluoylchloride; said 2-amino-2,3-dimethylbutyramide is the racemic 2,3-dimethyl-butyramide; and said compound is (±)-6-(4-isopropyl-4-methyl-5-oxo-2-imidazolin-2-yl)-m-toluic acid; and wherein said toluoyl chloride is para toluoylchloride; said 2-amino-2,3-dimethylbutyramide is (+); and said compound is (+)-6-(4-isopropyl-4-methyl-5-oxo-2-imidazolin-2-yl)-m-toluic acid.

5. A process for the preparation of a mixture (+)-6-(4-isopropyl-4-methyl-5-oxo-2-imidazolin-2-yl)-m-toluic acid and (+)-2-(4-isopropyl-4-methyl-5-oxo-2-imidazolin-2-yl) p-toluic acid, said process characterized by: reacting 4-methylphthalic anhydride with (+)-2-amino-2,3-dimethylbutyramide; and cyclizing said resultant components with sodium hydroxide.

**Revendications**

1. Composé choisi dans le groupe constitué par : le (+)-2-(4-isopropyl-4-méthyl-5-oxo-2-imidazoline-2-yl)-p-toluate de méthyle, ayant une rotation d'environ $[\alpha]_D^{25}$ = +1,06° (C = 0,026 g/ml de THF) et un point de fusion de 138°C-140°C ; le (+)-6-(4-isopropyl-4-méthyl-5-oxo-2-imidazoline-2-yl)-m-toluate de méthyle, ayant une rotation d'environ $[\alpha]_D^{25}$ = +3,29° (C = 0,029 g/ml de THF) et un point de fusion de 137°-140°C ; l'acide (+)-2-(4-isopropyl-4-méthyl-5-oxo-2-imidazoline-2-yl)-p-toluique ayant une rotation d'environ $[\alpha]_D^{25}$ = +6,09° (C = 0,027 g/ml de THF) ; l'acide (+)-6-(4-isopropyl-4-méthyl-5-oxo-2-imidazoline-2-yl)-m-toluique ayant une rotation d'environ $[\alpha]_D^{25}$ = +5,37° (C = 0,042 g/ml de THF) ; l'acide (±)-2-(4-isopropyl-4-méthyl-5-oxo-2-imidazoline-2-yl)-p-toluique ; l'acide (±)-6-(4-isopropyl-4-méthyl-5-oxo-2-imidazoline-2-yl)-m-toluique, ayant un point de fusion de 185°-187°C ; ou un sel d'addition d'acide de ceux-ci ; ou un sel de métal alcalin, d'ammonium ou d'ammonium aliphatique de ceux-ci.

2. Procédé de maîtrise des espèces de plantes indésirables, qui comprend l'application au feuillage des plantes ou au sol contenant les graines, les plantules ou les organes de propagation des plantes, d'une quantité herbicide efficace d'un composé choisi dans le groupe constitué par :
le (+)-2-(4-isopropyl-4-méthyl-5-oxo-2-imidazoline-2-yl)-p-toluate de méthyle, ayant une rotation d'environ $[\alpha]_D^{25}$ = +1,06° (C = 0,026 g/ml de THF) et un point de fusion de 138°C-140°C ; le (+)-6-(4-isopropyl-4-méthyl-5-oxo-2-imidazoline-2-yl)-m-toluate de méthyle, ayant une rotation d'environ $[\alpha]_D^{25}$ = +3,29° (C = 0,029 g/ml de THF) et un point de fusion de 137°-140°C ; l'acide (+)-2-(4-isopropyl-4-méthyl-5-oxo-2-imidazoline-2-yl)-p-toluique ayant une rotation d'environ $[\alpha]_D^{25}$ = +6,09° (C = 0,027 g/ml de THF) ; l'acide (+)-6-(4-isopropyl-4-méthyl-5-oxo-2-imidazoline-2-yl)-m-toluique ayant une rotation d'environ $[\alpha]_D^{25}$ = +5,37° (C = 0,042 g/ml de THF) ; l'acide (±)-2-(-4-isopropyl-4-méthyl-5-oxo-2-imidazoline-2-yl)-p-toluique ; l'acide (±)-6-(4-isopropyl-4-méthyl-5-oxo-2-imidazoline-2-yl)-m-toluique ; ou un sel d'addition d'acide de ceux-ci ; ou un sel de métal alcalin, d'ammonium ou d'ammonium aliphatique de ceux-ci.

3. Procédé de préparation des formes dextrogyres et des mélanges racémiques de chacun des régio-isomères méta et para des composés répondant aux formules développées :

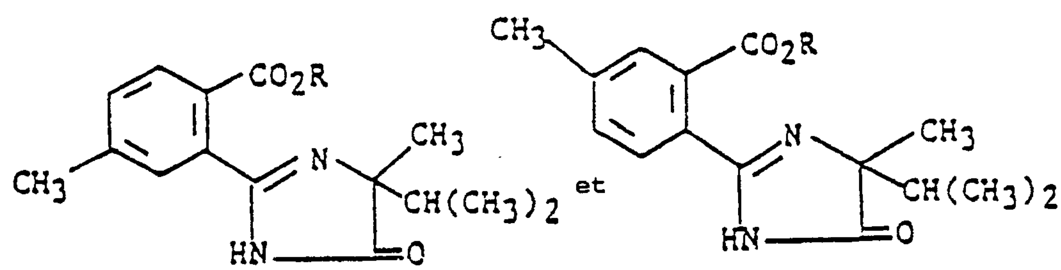
et

dans lesquelles R est H, ce procédé étant caractérisé par : la réaction du chlorure de p-toluoyle ou du chlorure de m-toluoyle avec un équivalent de la forme racémique ou (+) du 2-amino-2,3-diméthylbutyramide pour former le N-(1-carbamoyl-1,2-diméthylpropyl) toluamide ; la cyclisation de ce toluamide avec deux équivalents d'une base forte telle que l'hydrure de sodium pour former la 4-isopropyl-4-méthyl-2-tolyl-2-imidazoline-5-one ; la métallation de ladite 2-imidazoline-5-one avec 2,2 équivalents de sec-butyllithium ; et le traitement du mélange réactionnel ainsi formé avec du $CO_2$ pour former l'acide toluique désiré.

4. Procédé selon la revendication 3, dans lequel ledit chlorure de toluoyle est le chlorure de méta-toluoyle ; ledit 2,3-diméthylbutyramide est le 2-amino-2,3-diméthylbutyramide racémique ; et ledit composé est l'acide (±)-2-(4-isopropyl-4-méthyl-5-oxo-2-imidazoline-2-yl)-p-toluique ; dans lequel ledit chlorure de toluoyle est le chlorure de méta-toluoyle ; ledit 2-amino-2,3-diméthylbutyramide est l'isomère (+) ; et ledit composé est l'acide (+)-2-(4-isopropyl-4-méthyl-5-oxo-2-imidazoline-2-yl)-p-toluique ; dans lequel ledit chlorure de toluoyle est le chlorure de para-toluoyle ; ledit 2-amino-2,3-diméthylbutyramide est le 2,3-diméthylbutyramide racémique ; et ledit composé est l'acide (±)-6-(4-isopropyl-4-méthyl-5-oxo-2-imidazoline-2-yl)-toluique ; et dans lequel ledit chlorure de toluoyle est le chlorure de para-toluoyle ; ledit 2-amino-2,3-diméthylbutyramide est l'isomère (+) ; et ledit composé est l'acide (+)-6-(4-isopropyl-4-méthyl-5-oxo-2-imidazoline-2-yl)-m-toluique.

5. Procédé pour la préparation d'un mélange d'acide ( + )-6-(4-isopropyl-4-méthyl-5-oxo-2-imidazoline-2-yl)-m-toluique et d'acide ( + )-2-(4-isopropyl-4-méthyl-5-oxo-2-imidazoline-2-yl)-p-toluique, ledit procédé étant caractérisé par la réaction de l'anhydride 4-méthylphtalique avec le ( + )-2-amino-2,3-diméthylbutyramide ; et la cyclisation des composants obtenus avec l'hydroxyde de sodium.

## Ansprüche

1. Verbindung, ausgewählt aus der Gruppe bestehend aus: Methyl-( + )-2-(4-isopropyl-4-methyl-5-oxo-2-imidazolin-2-yl)-p-toluat, mit eine Drehung von etwa [alpha]$_D^{25}$ + 1,06° (C = 0,026 g/ml THF) und einem Schmelzpunkt von 138 bis 140° C; Methyl-( + )-6-(4-isopropyl-4-methyl-5-oxo-2-imidazolin-2-yl)-m-toluat, mit einer Drehung von etwa [alpha]$_D^{25}$ + 3,29° (C = 0,029 g/ml THF) und einem Schmelzpunkt von 137 bis 140° C; ( + )-2-(4-Isopropyl-4-methyl-5-oxo-2-imidazolin-2-yl)-p-toluolsäure, mit einer Drehung von etwa [alpha]$_D^{25}$ + 6,09° (C = 0,027 g/ml THF); ( + )-6-(4-Isopropyl-4-methyl-5-oxo-2-imidazolin-2-yl)-m-toluolsäure, mit einer Drehung von etwa [alpha]$_D^{25}$ + 5,37° (C = 0,042 g/ml THF); (±)-2-(4-Isopropyl-4-methyl-5-oxo-2-imidazolin-2-yl)-p-toluolsäure; (±)-6-(4-Isopropyl-4-methyl-5-oxo-2-imidazolin-2-yl)-m-tuluolsäure mit einem Schmelzpunkt von 185 bis 187° C; oder das Säureadditionssalz derselben; oder das Alkalimetallsalz, Ammoniumsalz oder aliphatische Ammoniumsalz derselben.

2. Verfahren zur Kontrolle von unverwünschten Pflanzenspecies, wobei man auf das Blattwerk der Pflanzen oder den Boden, der Samen, Sämlinge oder Fortpflanzungsorgane der Pflanzen enthält, eine herbizidwirksame Menge einer Verbindung appliziert, ausgewählt aus der Gruppe bestehend aus: Methyl-( + )-2-(4-isopropyl-4-methyl-5-oxo-2-imidazolin-2-yl)-p-toluat, mit eine Drehung von etwa [alpha]$_D^{25}$ + 1,06° (C = 0,026 g/ml THF) und einem Schmelzpunkt von 138 bis 140° C; Methyl-( + )-6-(4-isopropyl-4-methyl-5-oxo-2-imidazolin-2-yl)-m-toluat, mit einer Drehung von etwa [alpha]$_D^{25}$ + 3,29° (C = 0,029 g/ml THF) und einem Schmelzpunkt von 137 bis 140° C; ( + )-2-(4-Isopropyl-4-methyl-5-oxo-2-imidazolin-2-yl)-p-toluolsäure, mit einer Drehung von etwa [alpha]$_D^{25}$ + 6,09° (C = 0,027 g/ml THF); ( + )-6-(4-Isopropyl-4-methyl-5-oxo-2-imidazolin-2-yl)-m-toluolsäure, mit einer Drehung von etwa [alpha] + 5,37° (C = 0,042 g/ml THF); (±)-2-(4-isopropyl-4-methyl-5-oxo-2-imidazolin-2-yl)-p-toluolsäure; (±)-6-(4-isopropyl-4-methyl-5-oxo-2-imidazolin-2-yl)-m-tuluolsäure mit einem Schmelzpunkt von 185 bis 187° C; oder das Säureadditionssalz derselben; oder das Alkalimetallsalz,Ammoniumsalz oder aliphatische Ammoniumsalz derselben.

3. Verfahren zur Herstellung der rechtsdrehenden und racemischen Mischungen von jeweils den meta und para Regioisomeren von Verbindungen mit der Strukturformeln:

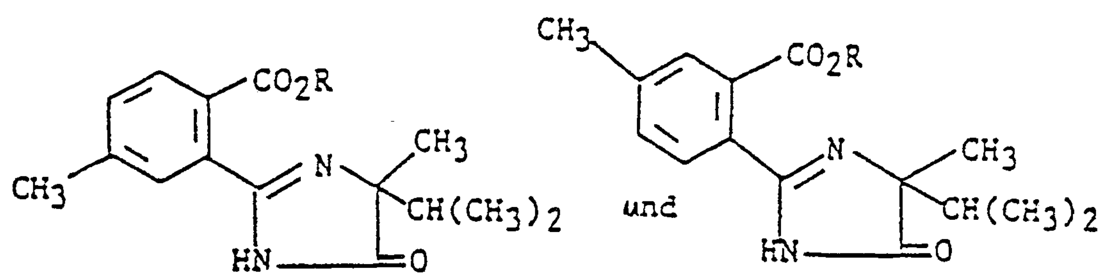

und

wobei R für H steht, wobei das Verfahren dadurch gekennzeichnet ist, daß man entweder p-Toluoylchlorid oder m-toluoylchlorid mit einem Äquivalent von racemischem oder ( + )-2-Amino-2,3-dimethylbutyramid umsetzt, um das N-(1-carbamoyl-1,2-dimethylpropyltoluamid zu bilden; das erwähnte Toluamid mit zwei Äquivalenten einer starken Base, wie beispielsweise Natriumhydrid cyclisiert, um ein 4-Isopropyl-4-methyl-2-toluoyl-2-imidazolin-5-on zu erhalten; dieses 2-Imidazolin-5-on mit 2,2 Äquivalenten sek.-Butyllithium metalliert; und das so gebildete Reaktionsgemisch mit $CO_2$ behandelt, um die gewünschte Toluolsäure zu erhalten.

4. Verfahren gemäß Anspruch 3, wobei es sich bei dem Toluoylchlorid um das meta-Toluoylchlorid handelt; bei dem 2,3-Dimethylbutyramid um das racemische 2-Amino-2,3-dimethylbutyramid handelt und bei der erwähnten Verbindung um (±)-2-(4-Isopropyl-4-methyl-5-oxo-2-imidazolin-2-yl)-p-toluolsäure handelt; wobei es sich bei dem Toluoylchlorid um das meta-Toluoylchlorid handelt; das 2-Amino-2,3-dimethylbutyramid das ( + )-Isomere ist; und die erwähnte Verbindung die ( + )-2-(4-Isopropyl-4-methyl-5-oxo-2-imidazolin-2-yl)-p-toluolsäure ist; wobei es sich bei dem Toluoylchlorid um para-Toluoylchlorid handelt; bei dem 2-Amino-2,3-dimethylbutyramid um das racemische 2,3-Dimethylbutyramid handelt; und die erwähnte Verbindung (±)-6-(4-Isopropyl-4-methyl-5-oxo-2-imidazolin-2-yl)-m-toluolsäure ist; und wobei es sich bei dem Toluoylchlorid um para-Toluoylchlorid handelt; bei dem 2-Amino-2,3-dimethylbutyramid um das (+)-Isomere handelt; und die erwähnte Verbindung (+)-6-(4-Isopropyl-4-methyl-5-oxo-2-imidazolin-2-yl)-m-toluolsäure ist.

5. Verfahren zur Herstellung einer Mischung von (+)-6-(4-Isopropyl-4-methyl-5-oxo-2-imidazolin-2-yl)-m-toluolsäure und (+)-2-(4-Isopropyl-4-methyl-5-oxo-2-imidazolin-2-yl-p-toluolsäure, wobei das Verfahren dadurch gekennzeichnet ist, daß man 4-Methylphthalsäureanhydrid mit (+)-2-Amino-2,3-dimethylbutyramid umsetzt und die resultierenden Komponenten mit Natriumhydroxid cyclisiert.